# EUROPEAN PATENT APPLICATION

(11) **EP 1 279 405 A1**
(43) Date of publication of application: **29.01.2003**
(21) Application number: 01917574.4
(22) Date of filing: 28.03.2001
(51) Int. Cl.: A61K 47/48, A61K 38/02, A61K 39/395, A61K 38/36, A61K 38/20, A61K 38/21, A61K 38/19, A61K 38/18, A61K 38/44, A61K 47/34

(54) **DRUGS RETAINED IN TARGET TISSUE OVER LONG TIME**

(30) Priority: 30.03.2000 JP 2000093775
(71) Applicant: Ajinomoto Co., Inc., Tokyo 104-8315 (JP)
(72) Inventor: SATO, Haruya, Central Research Laboratories, Kawasa-shi, Kanagawa 210-0801 (JP); HAYASHI, Eiko, Central Research Laboratories, Kawasaki-shi, Kanagawa 210-0801 (JP); SHIRAE, Hideyuki, Ajinomoto Co., Inc., Tokyo 104-8315 (JP)
(74) Representative: Nash, David Allan
(86) International application number: JP0102604
(87) International publication number: WO01074399

(57) **Abstract**

Provided are a polyethylene glycol-bound ligand in which a polyethylene glycol chain is bound to a ligand having a binding affinity to a specific receptor or a protein such as an antigen or the like present on a cell membrane of a target tissue and having an ability to avoid internalization (incorporation) into cells and a medicament in which a drug such as a physiologically active protein or the like is bound to the polyethylene glycol chain thereof.

A novel ligand which is accumulated at a high concentration around a target tissue and has a high retention property in blood and an excellent medicament in which a drug effective for the target tissue, such as a physiologically active protein or the like, is introduced therein can be provided.

## Description

### Technical Field

The present invention relates to a polyethylene glycol-bound ligand in which polyethylene glycol is introduced into a ligand having a binding affinity to a specific receptor or a protein such as an antigen or the like in a target tissue and having an ability to avoid internalization into cells, a novel medicament (medicine) in which a drug is introduced in a polyethylene glycol chain of the ligand, and a medical composition containing (comprising) the same as an effective ingredient.

### Technical Background

In recent years, various proteinous medicaments have been developed through production by a gene recombination technology or a large-scale (mass) cell culture method. The current situation is that these medicaments have been actually used with two defects that (1) administration in a high dose is required because they are easily degradable in vivo with enzymes and a retention in blood is low and (2) because of its low targetability with various physiological activities, there is a problem of the side effects of proteins due to the distribution around organs or cells other than target ones. For example, interferon α (IFNα) or β, which is an agent for treating chronic hepatitis C, is administered in a high dose three times a week over a long term to show an antiviral effect to virally infected hepatic parenchymal cells. Meanwhile, since various actions such as cell growth inhibition, immune response control, MHC antigen expression control and the like are expressed by binding to IFN receptors on the normal cells present generally in a whole body, it has involved problems of side effects such as pyrexia, reduction of platelets or granulocytes, interstitial pneumonia, abnormal thyroid function and the like.

Since these side effects and attending a hospital three times a week greatly influence QOL of patients, a long-term retention-type polyethylene glycol-IFNα modified with polyethylene glycol has been developed in recent years as an approach to solve these problems. In this polyethylene glycol-IFNα, polyethylene glycol has been introduced, with the result that the half life in blood has been prolonged and administration once a week has shown the same antiviral effect as the past administration three times a week. However, it has been problematic in that since a target orientation is low and it is retained in the whole body at a high concentration, thereby causing side effects such as pyrexia and the like to be increased in comparison to the past unmodified products.

On the other hand, with respect to interleukin-2 expected as an immunotherapeutic agent of cancers, the development of medications (medicines) of the polyethylene glycol modified substance was attempted. However, because of a high toxicity caused by various functions thereof, the development has been currently stopped.

Thus, the past modification of proteinous medicaments (medicines) with polyethylene glycol has indeed advantages of such as reduction of the dose or the number of administrations because the retention of the drug in the target site is surely enhanced. However, they are problematic in that the drug is also retained in organs other than target ones and the side effects thereof are therefore the same as, or rather higher than, those of unmodified products.

In order to solve these problems, the present inventors of this application succeeded in using, as a liver recognition element, an artificial ligand (refer to Japanese Patent Kokai Publication JP-A-5-202085) having an appropriate binding affinity to an asialoglycoprotein receptor specifically expressed in hepatic parenchymal cells and also being hard of internalization (incorporation), directly binding this artificial ligand to interleukin-2, one of proteinous medicaments (medicines) to form an artificial ligand-modified proteinous medicines and accumulating the proteinous medicine in the liver upon using the same to increase the pharmaceutical effect locally in the liver (refer to WO 98/13381). Although this artificial ligand-modified proteinous medicine enabled the liver accumulation of the physiologically active protein and the expression of its pharmaceutical function through the general administration, it was not satisfactory enough with respect to the long-term retention of the drug and the long-term persistence of the pharmaceutical effect. Under these circumstances, the present inventors of this application synthesized an artificial ligand and polyethylene glycol-modified proteinous medicament (medicine) with one molecule of an artificial ligand and one molecule of a polyethylene glycol introduced respectively in a fusion protein having a base sequence of a transglutaminase in the N-terminus of interleukin-2. Regarding the artificial ligand and polyethylene glycol-modified proteinous medicine, the modified proteinous medicine having a synergistic effect of the accumulation in the liver and the retention in blood showed that it was present specifically in the liver at a high concentration for a long period of time owing to the synergistic effect of the accumulation in the liver and the retention in blood. However, since the two different elements were bound with separate transglutaminases, it suffered the problems that the production step was intricate and a recovery rate of the final purified product was approximately 1% and quite low, as well as the problems that it was not easy to identify and prove the respective binding sites of the two elements and it was difficult to prove constant qualities as a medicine.

### Problems to be solved by the Invention

The present invention aims to provide a novel ligand which can be accumulated around a specific receptor on a cell membrane at a high concentration owing to a binding affinity to the specific receptor by suppressing its disappearance at the initial stage of the administration as much as possible and which can be retained around the specific receptor in the target tissue for a long time due to a high retention in blood. Further, it aims to provide a medicament which ensures fixed qualities as a medicament that enables accumulation and long-term retention of a drug around a receptor by introducing the novel ligand into a drug such as a physiologically active protein or the like to be desirably accumulated around specific cells in a target tissue, and a medical composition containing the medicament as an effective (active) ingredient.

### Disclosure of the Invention

The present inventors have assiduously conducted investigations to solve the foregoing problems, and have consequently completed the present invention by using, as a ligand for accumulating a drug in a target tissue at a high concentration, a ligand having a binding affinity to a specific receptor or a protein such as an antigen or the like present on a cell membrane of a target tissue and having an ability to avoid internalization (incorporation) into cells, binding a polyethylene glycol chain thereto to form a polyethylene glycol-bound ligand and introducing a drug in the polyethylene glycol chain of this ligand.

That is, the present invention is a polyethylene glycol-bound ligand in which a polyethylene glycol chain is bound to a ligand having a binding affinity to a specific receptor or a protein such as an antigen or the like present on a cell membrane of a target tissue and having an ability to avoid internalization (incorporation) into cells. Further, the present invention is a medicament (medicine) containing (comprising) a polyethylene glycol-bound ligand derivative in which a drug is bound to the polyethylene glycol chain of the polyethylene glycol-bound ligand, or a medical composition containing (comprising) the medicament as an effective ingredient.

### Brief Description of the Drawings

[Fig. 1]

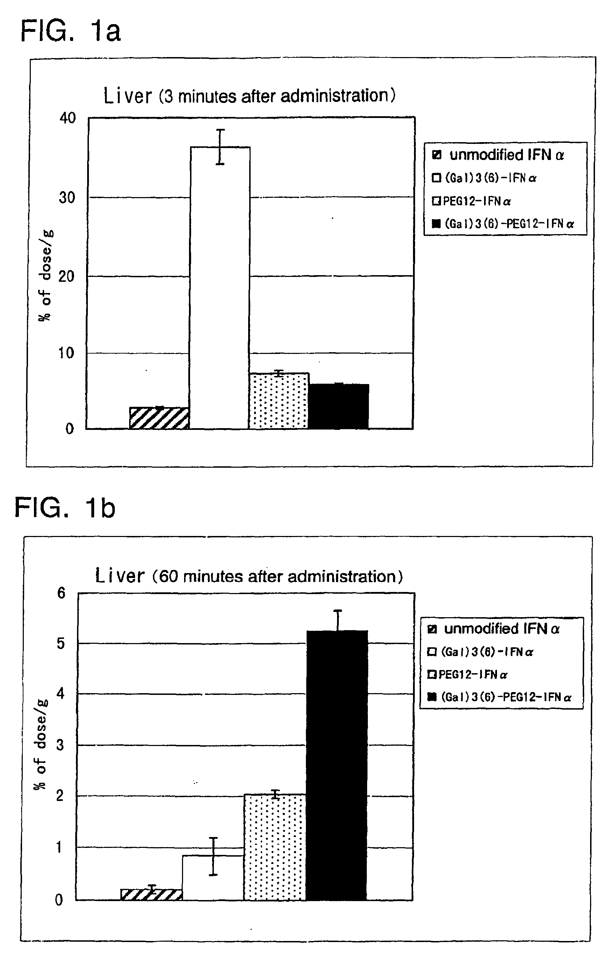

   Fig. 1 shows the results of an in-vivo behavior in Example 9, indicating a concentration of accumulation in the liver.
   Fig. 1a: 3 minutes after administration; Fig. 1b: 60 minutes after administration.
[Fig. 2]

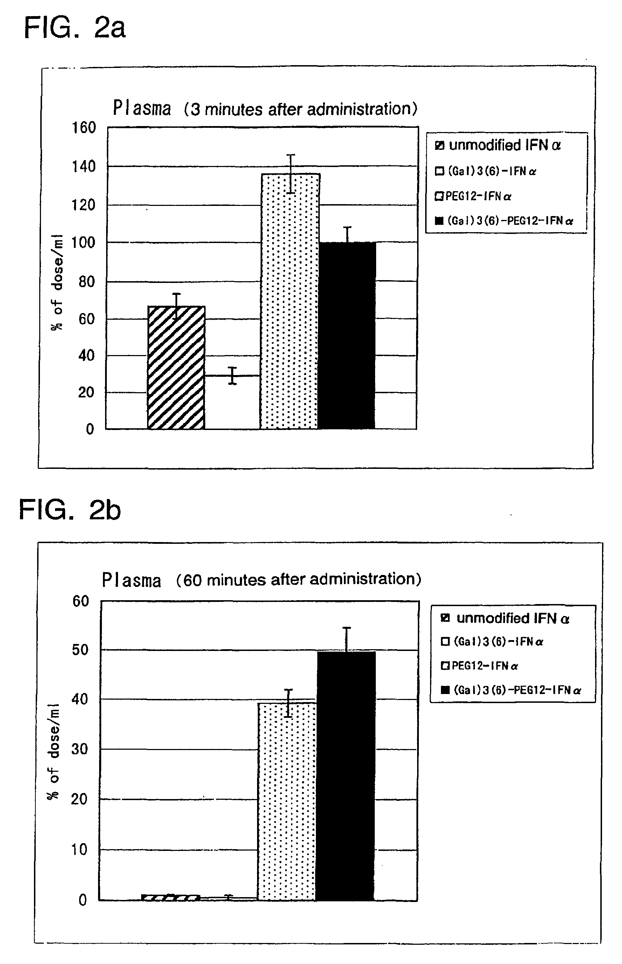

   Fig. 2 shows the results of an in-vivo behavior after intravenous administration to mice in Example 9, indicating a concentration in plasma.
   Fig. 2a: 3 minutes after administration; Fig. 2b: 60 minutes after administration.

In the foregoing drawings, four bar graphs all show unmodified IFNα, (Gal)₃(6)-IFNα, PEG12-IFNα and (Gal)₃(6)-PEG12-IFNα respectively from left.

### Embodiments

A polyethylene glycol-bound ligand in which a polyethylene glycol chain is bound to a ligand having a binding affinity to a specific receptor or a protein such as an antigen or the like present on a cell membrane of a target tissue and having an ability to avoid internalization (incorporation) into cells in the present invention exhibits a function, in actual use, especially in use as a medicament (medicine), by being bound to a drug of which the activity has been so far known. With respect to the molecular weight, a molecular weight of a drug in a medicament in which a drug is bound to a polyethylene glycol chain of the polyethylene glycol-bound ligand actually used as a medicament is preferably 60,000 or more for elimination in the kidney. However, a molecular weight of not more than 60,000 is also available. For this purpose, a molecular weight of polyethylene glycol used in the production may properly be determined from a molecular weight of a drug used and a molecular weight of a ligand used. It is preferably 5 kDa or more.

The target tissue of the present invention is an organ constituting a living body. It is not particularly limited so long as it becomes a target for accumulating a desired drug at a high concentration for therapy. Examples thereof can include a liver, a kidney, a bone marrow, a pancreas and the like.

The specific receptor or the protein such as the antigen or the like present on the cell membrane of the target tissue in the present invention has an affinity to the ligand in the present invention. However, it is preferable that the specific receptor or the protein such as the antigen or the like present on the cell membrane does not have an affinity to the drug. Further, for formation of the medicament intended by the present invention, the receptor is preferably specific to the target tissue. However, it is not necessarily specific thereto, and it may be a receptor in which a ligand can be accumulated in a target tissue. For example, when the liver is a target, an asialoglycoprotein receptor (ASGP-R) or the like expressed in a large amount in hepatic parenchymal cells is preferable. Besides this receptor, for example, a mannose receptor quite selectively expressed in hepatic non-parenchymal cells, and an epidermal cell growth factor (EGF) receptor, an insulin receptor, a transferrin receptor, a folate receptor and the like having a low cell specificity in expression but a high receptor density are mentioned.

On the other hand, examples of a protein expressed on a cell membrane except the receptor can include, for example various transporters, antigens and the like.

When a ligand is present, which has a binding affinity to compounds, the expression of which in specific cancer cells is increased in comparison to normal cells except the specific receptor and the protein such as the antigen or the like and these compounds exist on the target cell membrane, they can also be a subject of the ligand in the present invention.

The ligand in the present invention is not particularly limited so long as it has the binding affinity to the specific receptor or the protein such as the antigen or the like present on the cell membrane of the target tissue and having the ability to avoid internalization (incorporation) into cells, contains a functional group capable of being bound to the polyethylene glycol chain and does not show side effects in vivo. Further, this ligand may be a ligand which acts itself as a drug. However, it is preferable that the ligand itself does not have a physiological activity. In this case, when the ligand has a binding affinity to a specific receptor present on a cell membrane of a target tissue, a ratio of a dissociation rate constant and an internalization (incorporation) rate constant of the ligand to the specific receptor (a dissociation rate constant of the ligand to the specific receptor / an internalization (incorporation) rate constant of the ligand to the specific receptor) can be 1 or more. Preferably, a certain affinity and a certain ability to avoid internalization (incorporation) are required, that is, a dissociation constant has to be 10 mM or less or an internalization rate constant has to be less than 0.1 min⁻¹.

Such a ligand can be obtained from the known ligands having a binding affinity to a specific receptor or a protein such as an antigen or the like present on a cell membrane of a target tissue, or the ligands resulting from screening to a desired specific receptor or a desired protein such as an antigen or the like, or can be obtained by screening from the ligands designed on the basis of a structure of a desired specific receptor or a desired protein such as an antigen or the like and formed through synthesis, by the following method (refer to Japanese Patent Application No.11-186761; WO01/02851).

Incidentally, when a ligand to a protein on a target cell membrane is artificially synthesized, one to the receptor is easier to design it than one to other expression proteins.

The screening method is specifically described. It includes a method which comprises incubating a ligand or a ligand-modified substance having a binding affinity to a specific receptor or a protein such as an antigen or the like (hereinafter referred to as a "receptor or the like") present on a cell membrane in the presence of free cells or culture cells expressing the receptor, then washing the ligand bound to the cell surfaces or the substance modified with the ligand, thereafter adding a labeled ligand to the receptor or the like, incubating the mixture at a low temperature so as to suppress internalization (incorporation) into cells and screening a ligand or a ligand-modified substance in which the binding amount of the labeled ligand to the cell surfaces is higher than the binding amount when adding the non-labeled substance of the ligand having the affinity itself, a method which comprises incubating a ligand or a ligand-modified substance having a binding affinity to a specific receptor or the like present on a cell membrane in the presence of free cells or culture cells expressing the receptor or the like, then washing the ligand or the ligand-modified substance bound to the cell surfaces with a buffer containing a chelating agent or an acid buffer, thereafter adding a labeled ligand having an affinity to the receptor or the like, incubating the mixture at a low temperature so as to suppress internalization into cells and screening a ligand or a ligand-modified substance in which the binding amount of the labeled ligand to the cell surfaces is larger than the binding amount in adding a ligand in which a ratio of a dissociation rate constant and an internalization (incorporation) rate constant, that is a value of the dissociation rate constant/the internalization rate constant, after binding to the receptor or the like is at least 1, and so forth.

For example, as a ligand to the liver, a ligand to an asialoglycoprotein receptor expressed specifically in hepatic parenchymal cells and having a branched structure with galactose or N-acetylgalactosamine is mentioned. A structure (bone) used in the branching is preferably a structure branched with an acidic or basic amino acid. However, other compounds capable of making at least biantennarying (di-branching), such as trishydroxymethylaminomethane and the like, are available.

Examples of such compounds include compounds represented by the following general formula (I). 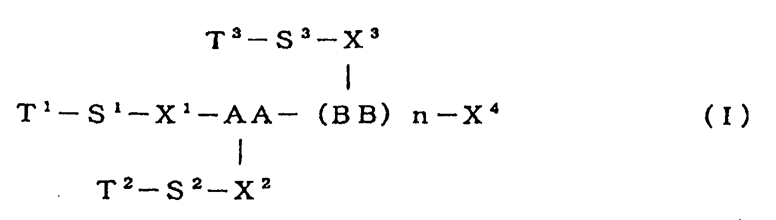 (wherein
T¹, T² and T³ which may be the same or different each represent galactose (Gal) or N-acetylgalactosamine (GalNAc),
S¹, S² and S³ which may be the same or different each represent (CH₂)ₚ or (CH₂CH₂O)_{q} in which p is an integer of 1 to 18 and q is an integer of 1 to 6,
AA and BB which may be the same or different each represent a basic amino acid or an acidic amino acid,
X¹, X² and X³ which may be the same or different each represent -CO- when bound to an amino group of an amino acid represented by AA or BB, or -NH- when bound to a carboxyl group of an amino acid represented by AA or BB,
X⁴ represents -COOH when bound to an amino group of an amino acid represented by AA or BB, or -NH₂ when bound to a carboxyl group of an amino acid represented by AA or BB,
n represents 0 or 1, and
when n is 1, amino acids represented by AA and BB may be amide-linked in any positions of the α-positions, the α-position and the γ-position, the γ-position and the α-position and the γ-positions).

With respect to these ligands, the ligands described in Japanese Patent Kokai Publication JP-A-202085 may be used.

Examples of the foregoing basic amino acid and acidic amino acid can include lysine, glutamic acid, aspartic acid and the like.

By the way, in the foregoing formula (I), a ligand represented by the following formula (II) is especially preferable.

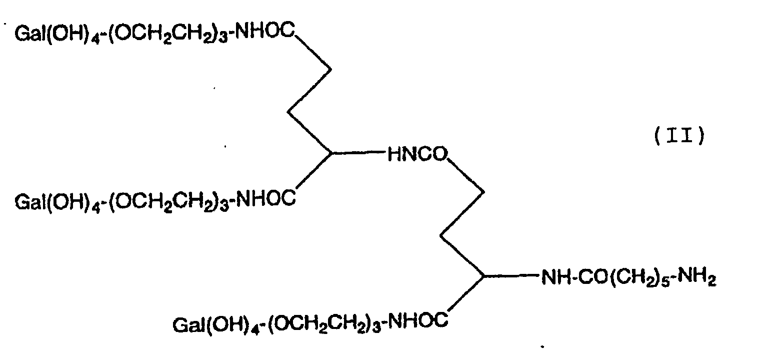

Polyethylene glycol used in the polyethylene glycol chain of the present invention has to have reactive functional groups in both ends in view of qualities of the present invention. The reactive functional groups here referred to may be functional groups capable of forming a chemical bonding with the ligand and the drug in the present invention. Examples thereof can include a hydroxyl group, an amino group, a carboxyl group, a vinyl group, an imidazole group, a mercapto group and the like . An alkylene group such as a methylene group, an ethylene group or the like, a sulfonyl group, a carbonyl group, an ether group, a disulfide group or the like may be present between these functional groups and the polyethylene glycol. For example, the following polyethylene glycol derivatives are available.

NH₂-CH₂CH₂-O-(CH₂CH₂O)ₙ-CH₂CH₂-NH₂

NH₂-CH₂-O-(CH₂CH₂O)ₙ-CH₂-NH₂

HOOC-O-(CH₂CH₂O)ₙ-COOH

HOOC-CH₂CH₂-COO-(CH₂CH₂O)ₙ-CO-CH₂CH₂-COOH

HOOC-CH₂-O-(CH₂CH₂O)ₙ-CH₂-COON

CH₂=CH-SO₂-(CH₂CH₂O)ₙ-CH₂CH₂-SO₂-CH=CH₂

(C₅NH₄)-S-S-(CH₂CH₂O)ₙ-S-S-(C₅NH₄)

HS-(CH₂CH₂O)ₙ-CH₂CH₂-SH

NH₂-CH₂CH₂-O-(CH₂CH₂O)ₙ-COOH

NH₂-CH₂CH₂-O-(CH₂CH₂O)ₙ-CH₂-COOH

NH₂-CH₂CH₂-O-(CH₂CH₂O)ₙ-CO-CH₂CH₂-COOH

NH₂-CH₂-O-(CH₂CH₂O)ₙ-COOH

NH₂-CH₂-O-(CH₂CH₂O)ₙ-CH₂-COOH

NH₂-CH₂-O-(CH₂CH₂O)ₙ-CO-CH₂CH₂-COOH

CH₂=CH-SO₂-(CH₂CH₂O)ₙ-COOH

CH₂=CH-SO₂-(CH₂CH₂O)ₙ-CH₂-COOH

CH₂=CH-SO₂-(CH₂CH₂O)ₙ-CO-CH₂CH₂-COOH

In the foregoing description, n represents an integer. The average molecular weight of polyethylene glycol and polyethylene glycol derivatives can be about (approximately) 1 kDa to 100 kDa. It is more preferable that the average molecular weight thereof is 3 kDa to 20 kDa and the range of the molecular weight is as small as possible.

The polyethylene glycol-bound ligand of the present invention can be produced by condensing polyethylene glycol derivatives and a ligand using an ordinary technique of organic synthesis such as an amide linkage, an ester linkage, an ether linkage, a disulfide linkage or the like depending on the types of functional groups thereof.

With respect to a specific example, a desired polyethylene glycol-bound ligand can be produced by, for example, introducing an alkylamine in which a terminal amino group is protected with a protective group such as Boc group or the like into one end of polyethylene glycol having carboxylic acids (carboxyl groups) in both ends, then introducing a ligand into an opposite end of the polyethylene glycol molecule and removing the protective group for the amino group. By the way, when the ligand is introduced into the opposite end of the polyethylene glycol molecule, another functional group may be protected for binding the polyethylene glycol to a desired site of the ligand.

Further, as required, it is also possible that a branched chain having two or more functional groups is introduced into one end of the polyethylene glycol and plural ligands are introduced.

The medicament (the polyethylene glycol derivative) in which the drug is bound to the polyethylene glycol chain of the polyethylene glycol-bound ligand in the present invention is one in which the ligand, the polyethylene glycol and the drug are bound in the order of ligand-polyethylene glycol-drug. For example, groups which become chains for binding them may be introduced therebetween.

Specific examples of the drug in the medicament in which the drug is bound to the polyethylene glycol chain of the polyethylene glycol-bound ligand in the present invention include physiologically active proteins such as plasma ingredients, e.g. immunoglobulins, blood coagulation factors and the like, cytokines, e.g. interleukins, interferons α/β/γ, tumor necrosis factor (TNF) and the like, cell growth factors, e.g. a hepatocyte growth factor (HGF), an epidermal cell growth factor and the like, and antioxidases, e.g. superoxide disumtase (SOD), catalase, thioredoxin and the like, and physiologically active peptides such as hormones, e.g. growth hormones, insulin and the like, and immunoreaction control factors, e.g. thymosin α and the like. The origin of such physiologically active proteins or physiologically active peptides is not particularly limited, and they may be derived from animals, plants or microorganisms. Further, proteins expressed and produced by incorporating genes or mutants of these proteins into E. coli, yeasts, Chinese hamster ovary cells or the like are also available. Still further, a chimera with other proteins is also available. Moreover, it is preferable that the physiologically active proteins or peptides in the present invention can be purified as much as possible before use to minimize the influence by co-existent proteins.

In addition, it is also possible to use, other than the physiologically active proteins, drugs of low-molecular compounds having a high toxicity and posing a problem of side effects owing to distribution in sites other than a target site, for example, antitumor agents such as adriamycin, mitomycin C, methotrexate and the like and antiviral agents such as azidothymidine (AZT), adenine arabinoside (ara-A), ribavirin and the like.

The low-molecular compounds having such a high toxicity can also be introduced into the polyethylene glycol-bound ligand of the present invention through natural proteins such as albumin, globulins and the like, various monoclonal antibodies, polysaccharides such as dextran, chitin, chitosan, inulin and the like, polyamino acids such as polylysine, polyglutamic acid and the like, synthetic polymers such as a divinyl ether maleic anhydride copolymer (DIVEMA), a styrene maleic anhydride copolymer (SMA), polyvinyl alcohol and the like, and lipid aggregate carriers such as liposome, lipid microspheres and the like, which can be used as carriers thereof.

For producing the medicament in which the drug is bound to the polyethylene glycol chain of the polyethylene glycol-bound ligand, it is possible that the polyethylene glycol-bound ligand is previously produced and the drug is bound to the polyethylene glycol chain thereof, or that polyethylene glycol is bound to the drug to produce a polyethylene-bound drug and the ligand is bound thereto.

In the present invention, the ligand, polyethylene glycol and the drug can be bound in a usual manner. The binding between them is preferably conducted through a covalent bond, which is not limited thereto, though. The binding can be conducted by a chemical method or by a method using an enzyme such as a transglutaminase or the like.

Further, the molar ratio thereof is not necessarily 1:1:1. A combination of ligand:polyethylene glycol:drug at a ratio of 2:1:1, 2:2:1, 3:1:1, 3:3:1 or the like is considered.

A method of binding the drug and the polyethylene glycol chain of the polyethylene glycol-bound ligand or the polyethylene glycol can include the following methods.

First, when the drug is a protein, it is preferable, as a medicine, that the structure thereof is uniform and its biological activity is not decreased. Therefore, it is possible that an alkyl amine is introduced into the polyethylene glycol chain or polyethylene glycol of the polyethylene glycol-bound ligand and reacted with the protein using a transglutaminase (refer to WO 96/06181 and WO 96/10089). In view of conducting selective site modification of a specific glutamine residue only, it is especially preferable to use a microbial transglutaminase described in the official gazette of WO 96/10089.

Specifically, a physiologically active protein or peptide, the polyethylene glycol-bound ligand and a transglutaminase, more preferably a microbial transglutaminase are reacted in an aqueous solution, more preferably with pH of approximately 7.5 at room temperature for 12 hours. The concentration ratio of the physiologically active protein or peptide and the polyethylene glycol- bound ligand is preferably in the range of 1:100 to 1:2,000. Further, the amount of the transglutaminase used is 0.01 to 1 unit per 1 nmol of the protein.

As another introduction method, it is also possible to use a method which comprises introducing SPDP (N-Succinimidyl-3-(2-pyridyldithio)propionate), SMPT (Succinimidyloxycarbonyl-α-methyl-α-(2-pyridyldithio)toluene), SMCC (Succinimidyl-4-(N-maleimidomethyl)cyclohexane-1-carboxylate) or the like into the polyethylene glycol chain of the polyethylene glycol-bound ligand or the polyethylene glycol and reacting the protein therewith to selectively modify only a Cys residue in the protein (refer to Goodson and Katre, 1990, Biothcnology 8, 343 - 346, and Benhar et al., J. Biol. Chem. 269, 13398 - 13404).

Moreover, in case of a protein of which the amino acid in the N-terminus is serine or threonine, a method in which an aminoxy derivative is specifically introduced into the amino group in the N-terminus by controlling reaction conditions such as a pH value and the like (refer to Bioconjugate Chem. 1996, 7, 38-44) and the like are mentioned.

In addition, the introduction can be also conducted by random modification, for example, a lysine residue side chain amino group modification method using a polyethylene glycol-bound ligand with a functional group introduced, such as a trichloro-s-triazine method, a carboxyimidazole method, a succinimidyl succinate method or the like, which is low in reaction selectivity, though.

Meanwhile, when the drug is a low-molecular compound, various methods such as a cyanogen bromide method, a periodate oxidation method, a carbidiimide method, a glutaraldehyde method, a mixed acid anhydride method, an SPDP reagent method and the like are mentioned. Incidentally, in this case, easy decomposition for exhibiting a pharmaceutical activity within and without cells at good efficiency, namely easy separation of a prodrug-like ligand has also to be taken into consideration.

The binding of these low-molecular compounds and the polyethylene chain of the polyethylene glycol-bound ligand or the polyethylene glycol can be conducted as follows, for example.

A low-molecular compound having an amino group or a carboxyl group can be bound through an amide linkage. In case of a low-molecular compound having an amino group, a carboxyl group is introduced into the polyethylene glycol chain or the polyethylene glycol, and these compounds are condensed by an ordinary method used in the organic synthesis. On the other hand, in a low-molecular compound having a carboxyl group, an amino group is introduced into the polyethylene glycol chain or the polyethylene glycol, and these compounds are condensed by an ordinary method used in the organic synthesis.

Condensation can be conducted by a reaction under dehydro-condensation conditions, specifically at a reaction temperature of 0°C to room temperature for 1 to 24 hours in a solvent not participating in the reaction (for example, acetonitrile, dimethylformamide, methylene chloride, ethylene chloride and the like) in the presence of an appropriate catalyst (for example, 1-ethyl-3-(3'-dimethylaminopropyl)carbodiimide, N-hydroxysuccinimide, N,N'-dicyclohexylcarbodiimide, 1-hydroxybenzotriazole and the like).

A low-molecular compound having a hydroxyl group or a carboxyl group can be bound through an ester linkage. In a low-molecular compound having a hydroxyl group, a carboxyl group is introduced into the polyethylene glycol chain or the polyethylene glycol, and these compounds are condensed by an ordinary method used in the organic synthesis. On the other hand, in a low-molecular compound having a carboxyl group, such a procedure is unnecessary because a polyethylene glycol has a hydroxyl group.

Condensation can be conducted by a reaction under dehydro-condensation conditions, specifically in a solvent not participating in the reaction (for example, acetonitrile, dimethylformamide, methylene chloride, ethylene chloride and the like) in the presence of an appropriate catalyst (for example, 1-ethyl-3-(3'-dimethylaminopropyl)carbodiimide, N-hydroxysuccinimide, N,N'-dicyclohexylcarbodiimide, 1-hydroxybenzotriazole and the like) at a reaction temperature of 0°C to room temperature for 1 to 24 hours.

Further, a low-molecular compound having a hydroxyl group can be bound through an ether linkage.

Condensation can be conducted by introducing an active halogen group such as a chloro group, a bromo group and the like and a leaving group such as a tosyl group and the like into the low-molecular compound or the polyethylene glycol chain, and reacting the thus obtained substance with a compound to be bound thereto. Condensation can be conducted by, as required, treatment with a hydride reagent such as sodium hydride, potassium hydride or the like and a reaction in a solvent not participating in the reaction (for example, dimethylformamide, tetrahydrofuran and the like) at a reaction temperature of 0°C to 100°C for 1 to 48 hours.

Besides the foregoing, a low molecular compound and a polyethylene glycol chain of a polyethylene glycol-bound ligand or a polyethylene glycol can be bound by an ordinary method of the organic synthesis depending on the structure of the low-molecular compound using a disulfide linkage, a urethane linkage or the like. In these instances, a functional group for linkage as a spacer may be introduced, as required, for binding.

The medicament of the present invention can be used as a malignant tumor treating agent, an antiviral agent, an antiallergic agent, an immunomodulator, a circulatory function improving agent, an internal secretion function improving agent, an agent for treating or preventing diseases caused by protein abnormal expression or abnormal function or the like, depending on a drug bound to a polyethylene glycol-bound ligand, namely, a physiologically active protein or a low-molecular compound.

The medicament of the present invention can be prepared in any dosage form such as an intravenous or intramuscular injection, a rectal administration agent, an oleaginous suppository, a water-soluble suppository or the like, for example, according to the intended use. These various preparations can be produced by an ordinary method using, as required, an excipient, a bulking agent, a binder, a wetting agent, a disintegrant, a surfactant, a lubricant, a dispersing agent, a buffer agent, a preservative, a solubilizer, an antiseptic, an analgesic, a stabilizer and the like which are commonly used. Examples of the non-toxic additives available include lactose, fructose, glucose, starch, gelatin, magnesium carbonate, synthetic magnesium silicate, talc, magnesium stearate, methylcellulose, carboxymethylcellulose or salts thereof, gum arabic, polyethylene glycol, syrup, vaseline, glycerin, ethanol, propylene glycol, citric acid, sodium chloride, sodium sulfite, sodium phosphate and the like.

When the drug is a protein or a peptide, the administration method of the medicament (medication) in the present invention is preferably parenteral administration such as intravenous administration, subcutaneous administration, intramuscular administration, mucosal administration (application through mucosa) and the like. More preferable is intravenous administration. Further, in case of a low-molecular drug, oral administration is preferable, though its administration method is not limited. Still further, the dose is preferably lower than a saturation binding amount to a receptor for maximizing a target delivery efficiency. In comparison to an ordinary unmodified drug, the dose of the modified drug in the present invention can be decreased (at least 1/2).

Not only does the dose thereof vary with the drug used, but also it can properly be determined in consideration of the usage, the age, the sex and the degree of progression of the disease of the patient, and the like. When the medicament in the present invention, namely, the medicament in which the drug is bound to the polyethylene glycol chain of the polyethylene glycol-bound ligand, for example, polyethylene glycol-bound (Gal)3-modified IFNα, polyethylene glycol-modified IFNα and (Gal)3-modified IFNα is each intravenously administered to mice to measure the in-vivo behavior, the compound in the present invention, in comparison to the product modified with only the ligand ((Gal)3-modified IFNα), suppresses disappearance in the initial stage of administration by avoiding glomerular filtration in the kidney and also avoids disappearance in other organs in circulating blood to greatly improve a retention thereof in the liver. Moreover, with respect to the change in concentration in other organs, the compound in the present invention is retained at a lower concentration than the product modified with polyethylene glycol only (polyethylene glycol-modified IFNα).

Thus, it is accumulated and retained at a high concentration in a target tissue, and is retained at a low concentration in other organs. It is therefore suggested that a therapeutic index of IFNα can be increased.

### Preferred Embodiments

### (Example 1)

### Synthesis of a polyethylene glycol-bound ligand

A derivative in which a Boc-protected alkylamine (HOOC-(CH₂)₅-NHBoc) is introduced into one end of polyethylene glycol having carboxyl groups in both ends (average molecular weight 9,000 Da, HOOC-CH₂-(OCH₂CH₂)ₙ-O-CH₂-COOH) to become a substrate of a transglutaminase is synthesized. A glutamic acid-tribranched galactose ligand derivative obtained by removing a terminal alkylamine from (Gal)₃ formed by the method described in official gazette of WO 96/06181 and O-acetylating a hydroxyl group of galactose is introduced into the carboxyl group in the opposite end of this Boc alkylamine-introduced polyethylene glycol by an active ester method. The ligand, the terminal Boc group of the alkylamine-introduced polyethylene glycol and the OAc group are removed in order to obtain a polyethylene glycol-bound (Gal)₃.

### (Example 2)

### Synthesis of a medicament (medication) in which a drug is bound to a polyethylene glycol chain of a polyethylene glycol-bound ligand

The polyethylene glycol-bound (Gal)₃ obtained in Example 1 is reacted with human interferon α (IFNα) in the presence of a microbial transglutaminase by the method described in official gazette of WO 96/10089 to obtain an IFNα modified with polyethylene glycol-bound (Gal)₃ at a high recovery rate.

### (Example 3)

### Synthesis of a polyethylene glycol with an alkylamine introduced in one end

Synthesis of a polyethylene glycol with an alkylamine introduced in one end was conducted by the Sato et al. method (refer to Biochemistry, 35(40), 13072 (1996)). Dry DMF (55 ml) was added to 5.00 g of dicarboxylic acid-type polyethylene glycol (made by Nippon Oils and Fats Co., Ltd., average molecular weight 12,000 Da) (1), and the mixture was slightly heated for dissolution. The solution was cooled to 25°C, and 101 mg (0.75 mmol) of 1-hydroxybenzotriazole (HOBt) and 144 mg (0.75 mmol) of EDCI (1-ethyl-3-(3-dimethylaminopropyl)carbodiimide) were added thereto. After the mixture was stirred at the same temperature for 30 minutes, a DMF (5 ml) solution of 152 mg (0.75 mmol) of t-butyl N-(5-aminoamyl)carbamate (2) was added dropwise thereto. The reaction solution was heated at 35°C, and the reaction was conducted for 16 hours. HOBt (101 mg, 0.75 mmol) and 144 mg (0.75 mmol) of EDCI were added thereto. After the reaction was further conducted for 11 hours, 101 mg (0.75 mmol) of HOBt and 144 mg (0.75 mmol) of EDCI were added thereto. After the addition thereof, the reaction was further conducted for 11 hours, and the reaction solution was then concentrated to obtain 8.37 g of a crude product. The crude product was subjected to a column purification (silica gel 300 g, eluent chloroform/methanol = 95/5 → 92/8), and divided into a fraction of 0.83 g and a fraction of 1.46 g. The fraction of 0.83 g was subjected again to a column purification (silica gel 300 g, eluent chloroform/methanol = 95/5 → 50/50) to obtain 500 mg of a purified product (purified product 1). The fraction of 1.46 g was subjected again to a column purification (silica gel 300 g, eluent chloroform/methanol = 95/5 → 50/50) to obtain 1,058 mg of a purified product (purified product 2). The purified products were confirmed by ¹H-NMR such that only a half amount of a signal ascribable to an ethyl group adjacent to a terminal carboxyl group was shifted and a main signal ascribable to an ethylene glycol group in the PEG skeleton was retained.

### (Example 4)

### Synthesis of compound (5)

Trifluoroacetic acid (6.55 ml) was added dropwise to a dichloromethane (10 ml) solution of compound (4) (1.41 g, 0.81 mmol) synthesized by the method described in the official gazette of WO 01/02851 in an argon stream at 0°C for 30 minutes. After completion of the dropwise addition thereof, the reaction temperature was elevated up to 20°C, and the reaction was conducted for 30 minutes. The reaction solution was concentrated, and further subjected to azeotropy (azeotropic distillation) with ethanol three times to remove trifluoroacetic acid as much as possible. The resulting crude product was subjected to a column purification (silica gel 80 g, eluent chloroform → chloroform/methanol = 5/1) to obtain 1.35 g of a column purification product. This was subjected again to a column purification (silica gel 50 g, eluent chloroform → chloroform/methanol = 8/1) to obtain 457 mg of the compound (5) (yield 34%). The purified product was confirmed by ¹H-NMR such that a terminal Boc group was removed from the compound (4). Further, its purity was identified by a thin layer chromatography (TLC).

### (Example 5)

### Synthesis of (Gal(OAc)₄)₃-PEG(Boc)

In an argon stream, 670 g of compound (3), 51.4 mg (268 mmol) of EDCI and 35 ml of dry DMF were added, and 36.2 mg (268 mmol) of HOBt was further added thereto. To this solution was added dropwise a dry DMF (30 ml) solution of 220 mg (134 mmol) of compound (5). The temperature of the reaction solution was elevated, and the reaction was conducted at 33°C for 14 hours. The reaction solution was concentrated to obtain 1.08 g of a crude product. This was subjected to a column purification (silica gel 100 g, eluent chloroform/methanol = 95/5 → 93/7) to obtain 697 mg of (Gal(OAc)₄)₃-PEG(Boc). Likewise, 619 mg and 109 mg of (Gal(OAc)₄)₃-PEG(Boc) were obtained respectively from 610 mg and 102 mg of compounds (3). The structures of the purified products were confirmed from an identification of terminal Boc group and OAc group as a Gal protective group and further from a retention of a main signal ascribable to an ethylene glycol group in a PEG skeleton moiety as measured by ¹H-NMR (solvent CDCl₃). Further, the purities thereof were confirmed by TLC.

### (Example 6)

### Synthesis of (Gal(OAc)₄)₃-PEG

A dichloromethane (20 ml) solution of 20 ml of trifluoroacetic acid was added dropwise to a dry dichloromethane (100 ml) solution of 1.18 g of the (Gal(OAc)₄)₃-PEG(Boc) in an argon stream at 1°C for 1.2 hours. The temperature of the reaction solution was elevated to 14°C over a period of 1 hour for concentration. One hundred milliliters (100 ml) of dry ethanol was added for concentration, and a procedure of removing trifluoroacetic acid was repeated twice. The residue was dissolved in 10 ml of methanol, and the solution was neutralized with a 28 % NaOCH₃ methanol solution diluted to 10 times. The reaction solution was concentrated, and the resulting crude product was subjected to a column purification (silica gel 100 g, eluent chloroform/methanol = 95/5 → 1/1) to obtain 606 mg of a column-purified product. This was combined with 100 mg of a preliminary examination product, and the combination was repeatedly subjected to a column purification on the same scale four times to obtain 295 mg of (Gal(OAc)₄)₃-PEG. The purified product was confirmed by removal of the Boc group from the (Gal(OAc)₄)₃-PEG(Boc) through ¹H-NMR. Further, its purity was confirmed by TLC.

### (Example 7)

### Synthesis of (Gal)₃(6)-PEG

A 28% NaOCH₃ methanol solution (23 mg) was diluted with 15 ml of methanol and then added dropwise to a methanol solution of 290 mg of the (Gal(OAc)₄)₃-PEG at 3°C. After the dropwise addition thereof, the reaction was conducted at 15 to 21°C for 3.5 hours. Since a spot in the same site as that of the starting material was not decreased in the tracing of the reaction by TLC, 22 mg of a 28% NaOCH₃ methanol solution was diluted with 15 ml of methanol, and then added thereto dropwise. After the dropwise addition thereof, the reaction was conducted at 20 to 21°C for 1.5 hours. The reaction solution was cooled, and Dowex 50Wx8 cleaned with methanol was added in small portions thereto for neutralization. The reaction solution was filtered, and the filtrate was concentrated to dryness to obtain 250 mg of (Gal)₃(6)-PEG. The purified product was identified by confirming separation of the OAc group from the (Gal(OAc)₄)₃-PEG through ¹H-NMR. Further, the purity was confirmed by TLC.

### (Example 8)

### Preparation of (Gal)₃(6)-PEG-IFNα

One hundred micrograms (100 µg) of freeze-dried human IFNα(2b) (made by Seikagaku Kogyo K.K.) was dissolved in 744 µl of a 200 mM tris-hydrochloride buffer solution (pH 7.5), and 57.3 mg of (Gal)₃(6)-PEG obtained in Example 7 was added thereto. Approximately 0.7 U of a microbial transglutaminase was added to the reaction solution, and the mixture was incubated overnight at room temperature. The reaction solution was subjected to a SDS-PAGE (homogenious 20 (made by Pharmacia)) to confirm disappearance of unreacted IFNα. Then, unreacted (Gal)₃-PEG and M-TG were removed using "SEP-PAK" column (made by Millipore). The fraction obtained was concentrated with Speed Vac, and neutralized with PBS (+). Approximately 4.4 µg of (Gal)₃(6)-PEG-IFNα was obtained in this reaction.

The reactions described in Examples 3 to 8 were shown below using reaction formulas.

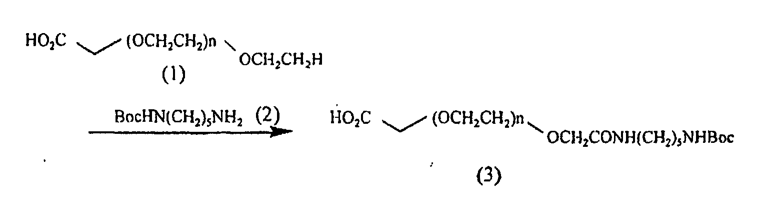

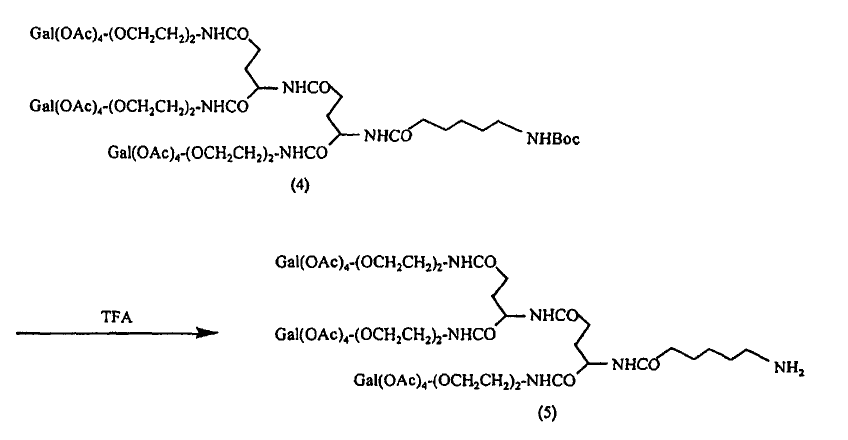

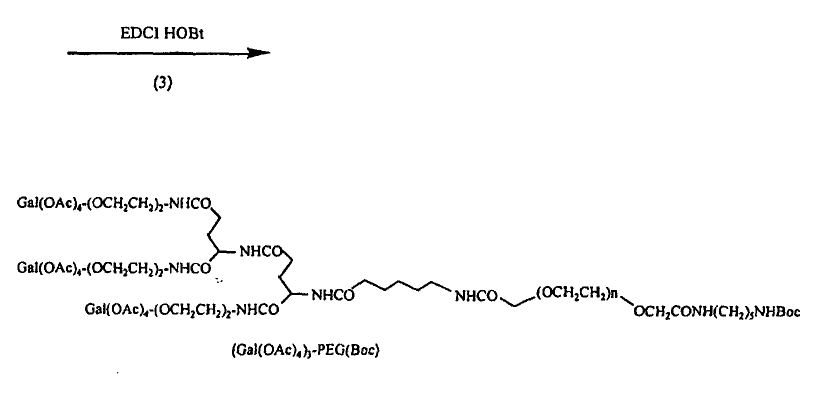

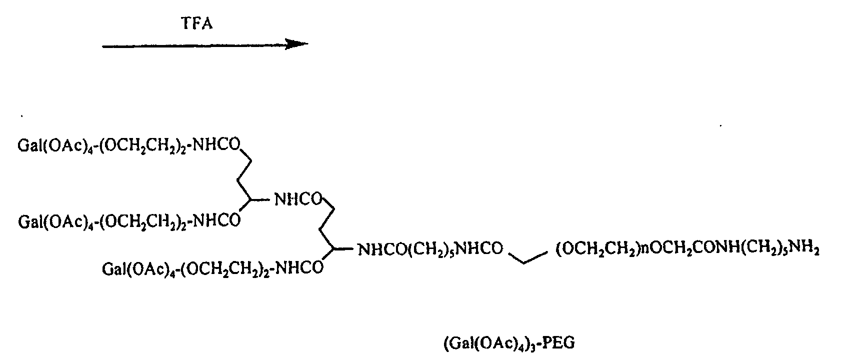

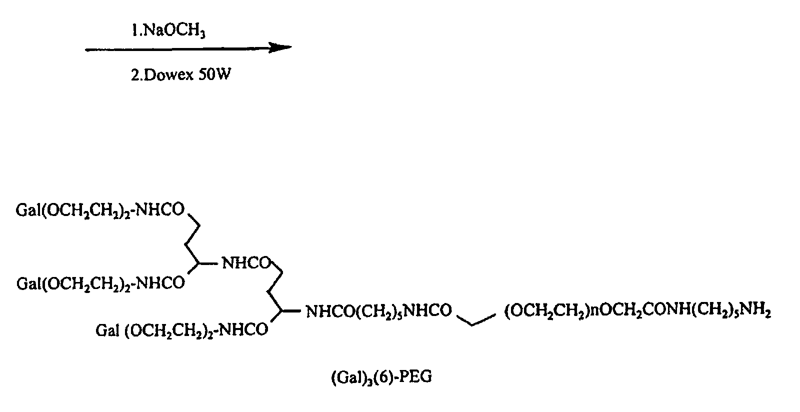

### (Example 9)

### In-vivo behavior of (Gal)₃(6)-PEG-IFNα

To the tail of each of 6-week-old male mice (C57BL/6, CRJ) were intravenously administered 19.9 µg/kg, calculated as IFNα, of (Gal)₃(6)-PEG-IFNα, PEG12-IFNα (PEG one molecule modified product having an average molecular weight of 12 kDa and obtained by chemically modifying one end methoxy PEG carboxylic acid made by Nippon Oils and Fats Co., Ltd. through an active ester method and then collecting only a one molecule modified product through a reversed phase HPLC (refer to Sato et al., Bioconjugate Chem., 11(4), 502 (2000)), (Gal)₃(6)-IFNα (prepared according to the method described in the official gazette of WO 01/02851) and unmodified IFNα, respectively. After the lapse of a fixed time (3 minutes or 60 minutes), blood sampling and sampling of the liver were conducted. By the way, the plasma was sampled in a usual manner, and a concentration in the specimen was measured by ELISA. The liver was homogenized by adding a homogenate buffer (MEM, 5% FBS, pH 7.2 to 7.4) in a 9-fold amount of the weight of the organ, and centrifuged at 3,000 rpm for 10 minutes. Subsequently, a supernatant was diluted with the same homogenate buffer, and a concentration in the specimen was measured by ELISA. Incidentally, ELISA was conducted using a Human ELISA IFNα kit (made by ENDOGEN). Consequently, as shown in Figs. 1 and 2, in the initial stage of the administration, (Gal)₃(6)-PEG-IFNα, like PEG12-IFNα, exhibited quite a high concentration in plasma (refer to Fig. 2a) and a low concentration of accumulation in the liver in comparison to (Gal)₃(6)-IFNα (refer to Fig. 1a). However, after 1 hour from the administration, the concentration of (Gal)₃(6)-PEG-IFNα in the liver was retained highest (refer to Fig. 1b), and it was approximately 2.6 times as high as that of PEG12-IFNα and approximately 6.2 times as high as (Gal)₃(6)-IFNα. This means that even after the lapse of a long time the concentration in the liver is retained high owing to the liver accumulation ability by the ligand and the retention effect by PEG. The foregoing results proved that an accumulation property (ability) in the liver and also a circulating (retention) property in the liver were imparted by providing the (Gal)₃(6) ligand as a liver recognition element and also PEG, and the usefulness of the novel ligand was clarified.

### Effects of the Invention

The dose of the medicament in the present invention can be decreased down to between 1/2 and 1/9 of the dose of the medicament with the unmodified product owing to the target accumulation and the long-term retention properties, and thus the present invention allows therapy with side effects reduced. Further, since the introduction of the polyethylene glycol-bound ligand to the drug is conducted by one step, the polyethylene glycol-bound ligand modified drug can be obtained at a high recovery rate by the simple production step. Still further, since the medicament of the present invention is a product modified with only one molecule of the polyethylene glycol-bound ligand, it is possible to easily prove the same by a peptide map or the like and ensure fixed qualities as a medicament (medicine).

## Claims

1. A polyethylene glycol-bound ligand in which a polyethylene glycol chain is bound to a ligand having a binding affinity to a specific receptor or a protein such as an antigen present on a cell membrane of a target tissue and having an ability to avoid internalization into cells.

2. The polyethylene glycol-bound ligand according to claim 1, wherein the ligand has a binding affinity to a specific receptor present on a cell membrane of a target tissue.

3. The polyethylene glycol-bound ligand according to claim 2, wherein a ratio of a dissociation rate constant and an internalization rate constant of the ligand through the specific receptor (the dissociation rate constant of the ligand through the specific receptor / the internalization rate constant of the ligand through the specific receptor) is 1 or more.

4. The polyethylene glycol-bound ligand according to claim 2 or 3, wherein a dissociation constant of the ligand to the specific receptor is 10 mM or less.

5. The polyethylene glycol-bound ligand according to claim 4, wherein the internalization rate constant of the ligand to the specific receptor is less than 0.1 min⁻¹.

6. The polyethylene glycol-bound ligand according to any one of claims 1 to 5, wherein the specific receptor present on the cell membrane of the target tissue is an asialoglycoprotein receptor (ASGP-R).

7. The polyethylene glycol-bound ligand according to any one of claims 1 to 6, wherein the target tissue is a liver.

8. A medicament **characterized in that** a drug is bound to the polyethylene glycol chain in the polyethylene glycol-bound ligand according to any one of claims 1 to 7, or a medical composition containing the medicament as an active ingredient.

9. The medicament or the medical composition containing the medicament as an active ingredient according to claim 8, wherein the drug is a physiologically active protein or a physiologically active peptide.

10. The medicament or the medical composition containing the medicament as an active ingredient according to claim 9, wherein the physiologically active protein is any one of immunoglobulins, blood coagulation factors, interleukins, interferons, a tumor necrosis factor, a hepatocyte growth factor (HGF), an epidermal cell growth factor, superoxide disumtase (SOD), catalase and thioredoxin.

11. The medicament or the medical composition containing the medicament as an active ingredient according to claim 9 or 10, wherein the physiologically active protein contains a glutamine residue and has a molecular weight of 1 x 10³ to 2 x 10⁵.

12. The medicament or the medical composition containing the medicament as an active ingredient according to claim 10, wherein the physiologically active protein is any one of interferon α, interferon β and interleukin-2.

13. The medicament according to claim 11 or 12, wherein the end of the polyethylene glycol chain of the polyethylene glycol-bound ligand is an amino group and which can be produced by reacting the amino group with a physiologically active protein in the presence of a transglutaminase to form an amide linkage, or the medical composition containing the medicament as an active ingredient.
